# EUROPÄISCHE PATENTSCHRIFT

(11) **EP 1 913 975 B1**
(45) Veröffentlichungstag und Bekanntmachung des Hinweises auf die Patenterteilung: **08.06.2011**
(21) Anmeldenummer: 07019008.7
(22) Anmeldetag: 27.09.2007
(51) Int. Cl.: A61Q 1/10, A61K 8/04, A61K 8/34, A61K 8/81, A61K 8/86, A61K 8/894

(54) **Kosmetische Zubereitung zum Färben der Augenlider und der Augenbrauen**
Cosmetic preparation for dyeing eyelashes and eyebrows
Préparation cosmétique destinée à la coloration des paupières et des cils

(30) Priorität: 20.10.2006 EP 06022005
(43) Veröffentlichungstag der Anmeldung: 23.04.2008
(73) Patentinhaber: Faber-Castell AG, 90546 Stein (DE)
(72) Erfinder: Lugert, Gerhard, Dr., 90431 Nürnberg (DE); Appel, Tatiana, 90522 Oberasbach (DE)
(74) Vertreter: Mörtel & Höfner

(56) Entgegenhaltungen:
- EP-A- 1 247 514
- JP-A- 2006 069 965
- US-A- 5 013 543

## Beschreibung

Die Erfindung betrifft eine kosmetische Zubereitung zum Färben der Augenlider und der Augenbrauen. Die Färbung der genannten Hautpartien erfolgte bisher hauptsächlich mit Hilfe relativ hoch viskoser Zubereitungen, die mit Hilfe von Bürsten, Pinseln u. dgl. aufgetragen wurden. Eine exakte Applikation ist auf die genannte Art nur schwer möglich. Außerdem ist ein mehrmaliges Aufnehmen von Zubereitung aus einem Vorratsbehälter erforderlich, um die gewünschte Hautpartie mit einem gleichmäßigen Farbauftrag zu versehen. Dabei ist es oft nicht zu vermeiden, dass Zubereitung von den Auftragsgeräten abtropft. Daher werden heute vielfach kapillare Auftragsgeräte verwendet, mit denen die erwähnten Handhabungsprobleme weitgehend gelöst sind. Problematisch bei kapillaren Auftragsgeräten ist, dass die Zubereitungen relativ niedrig viskos sein müssen, damit sie von einem Kapillarsystem eines Auftragsgeräts aus dessen Vorratsspeicher herausgefördert werden können. Sie müssen aber auch die an eine Zubereitung für den vorgesehenen Zweck gestellten Anforderungen erfüllen, d.h. sie sollen einen möglichst deckenden Farbauftrag ergeben, der eine für den Augenbereich ausreichende Permanenz aufweist und sich beim Abschminken leicht wieder entfernen lässt. Dies sind Eigenschaften, die mit niedrig viskosen Zubereitungen nur schwer zu erreichen sind, da diese wegen ihrer geringen Viskosität leicht in die Haut eindringen, was auch ihre Entfernung beim Abschminken erschwert. Niedrig viskose Zubereitungen neigen dazu in Fältchen und Hauttaschen einzudringen und darin zu verlaufen, so dass eingefärbte Hautareale mit einem einigermaßen glatten Rand nur schwer zu erzeugen sind. Es ergibt sich vielmehr ein eher ausgefranstes Erscheinungsbild.

Eine für den genannten Verwendungszweck konzipierte, aus JP-A-2006-6995 bekannte wässrige Zubereitung enthält als filmbildende Substanz eine Kunststoffdispersion auf Acrylat-Copolymerbasis, deren Feststoffanteil bezogen auf die Gesamtmenge der Zubereitung 2 % bis 20 % beträgt, 1 % bis 40 % eines Farbpigments, 3 % bis 20 % eines Feuchthaltemittels wie Glycol oder Glycerol, sowie 0,05 % bis 5 % eines polyethermodifizierten Polysiloxans (% = Gew.%).

Davon ausgehend ist es die Aufgabe der Erfindung, eine alternative kosmetische Zubereitung zum Färben der Augenlider und der Augenbrauen vorzuschlagen.

Diese Aufgabe wird durch eine wässrige Zubereitung nach Anspruch 1 gelöst. Diese enthält 3 Gew.% bis 35 Gew.% einer wässrigen Kunststoffdispersion auf Acrylat-Copolymerbasis mit einem Feststoffgehalt von 30 Gew.% bis 50 Gew.%, 1 Gew.% bis 20 Gew. % wenigstens eines Alkohols aus der Gruppe Ethanol, Propanol, 2 Gew.% bis 15 Gew.% wenigstens eines Feuchthaltemittels, 0,2 Gew.% bis 5 Gew.% Polyoxyethylen-glycerin-fettsäureester und/oder polyethermodifiziertes Polysiloxan, und ein Farbmittel, wobei darunter auch eine Mischung verschiedener Farbmittel zu verstehen ist, und weist eine Viskosität von weniger als 50 mPas (Brookfield, 25°C) auf.

Eine derartige Zubereitung lässt sich problemlos mit einem kapillaren Auftragsgerät auf ein Augenlid oder eine Augenbraue auftragen, wobei ein Verlaufen der Zubereitung in Fältchen und Hauttaschen vermieden ist, was vor allem der Kunststoffdispersion bzw. den darin enthaltenden Polymerpartikeln auf Acrylat-copolymerbasis und dem in der Zubereitung enthaltenen Alkohol (Ethanol, Propanol oder einer Mischung dieser Alkohole) zu verdanken ist. Die Zubereitung trocknet auf der Haut leicht ab, wodurch die Gefahr, den Farbauftrag etwa noch während der Applikation zu Verwischen verringert ist. Außerdem wird durch das schnelle Abtrocknen des Auftrags die Tendenz der Flüssigkeit, in Hautfältchen hinein zu fließen, weiter verringert. Die genannten Alkohole üben auf die Haut aufgrund von Feuchtigkeits- und Fettentzug eine gewisse versprödende Wirkung aus. Dieser Effekt wird jedoch erfindungsgemäß durch die Kombination von Alkohol mit Feuchthaltemitteln kompensiert wobei hier vorzugsweise 1,3-Butandiol, Propylenglykol und Glycerin zum Einsatz kommen. Glycerin wird aber vorzugsweise nicht alleine, sondern in Mischung mit einem der beiden anderen Feuchthaltemitteln eingesetzt. Die Kombination aus Feuchthaltemittel und wenigstens einem der genannten Alkohole hat aber auch im Hinblick auf die Lagerfähigkeit eines mit der Zubereitung gefüllten Applikators vorteilhafte Auswirkungen. Bei Zubereitungen der vorliegenden Art, die relativ leichtflüchtig sind, besteht die Gefahr, dass sie während der Lagerung bzw. des Nichtgebrauchs die Poren bzw. Kapillarkanäle eines mit der Zubereitung vollgesogenen Auftragselements durch Eintrocken verstopfen, so dass die Funktionsfähigkeit des Applikators eingeschränkt wird oder dieser ganz unbrauchbar wird. Es hat sich nun herausgestellt, dass dieser nachteilige Effekt bei Vorhandensein einer Kombination aus wenigstens einem der Alkohole Ethanol/Propanol und wenigstens einem Feuchthaltemittel zumindest verlangsamt ist, so dass längere Lagerzeiten möglich sind.

Um trotz der in der Zubereitung enthaltenen viskositätserhöhenden Substanzen, vor allem den genannten Kunststoffpartikeln und beispielsweise auch als Farbmittel zugesetzten Pigmenten für eine Anwendung in einem kapillaren Auftragsgerät ausreichende Viskositätswerte zu gewährleisten, ist Polyoxyethylen-glycerin-fettsäureester, Polyether-modifiziertes Polysiloxan oder vorzugsweise eine Mischung dieser Substanzen zugesetzt. Die Substanzen haben u.a. die Eigenschaft, dass sie einer Erhöhung der Viskosität der Zubereitung, die etwa durch die darin enthaltende Kunststoffdispersion bedingt ist, entgegenwirken. Dabei war es überraschend, dass die Zubereitung, insbesondere wenn beide Substanzen enthalten sind, dennoch nur eine geringe Tendenz aufweist, in Hautfältchen zu hinein zu fließen. Oberhalb eines Gehaltes von 5 % einer einzelnen Substanz oder einer Mischung ist dies nicht mehr gewährleistet. Unterhalb von 0,5 % ist eine merkliche viskositätsverringernde Wirkung kaum mehr zu erreichen.

Je nach den gewünschten Färbegraden beträgt der Anteil an Farbmitteln 0,5 Gew.% bis 30 Gew.%, wobei vorzugsweise Farbpigmente zum Einsatz kommen. Diese ergeben im Vergleich zu löslichen Farbstoffen in der Regel eine stärkere Färbung. Eine Anwendung in einem Kapillarsystem ist problemlos möglich, wenn eine Korngrößenverteilung der Pigmente von D90% < 10 µm eingehalten wird. Der Wassergehalt der Zubereitung reicht von 30 Gew.% bis 70 Gew.%.

Additive, etwa pflegende Mittel - wie Aloe Vera, Camilla Sinensis, Tocopherol oder Pantenol - und Konservierungsmitteln sind vorzugsweise auf insgesamt max. 5 Gew.% beschränkt.

### Beispiel 1:

| | |
|---|---|
| Schwarze Eyelinerflüssigkeit mit einer Viskosität von ca. 11mPa s (Brookfield, 20 °C) Wasser, dem. ¹⁾ | 27,0 Gew. % |
| Polyethylen-Glycerin-Stearinsäure-Ester (CAS 68553-11-7) | 2,0 Gew. % |
| Polyethermodifiziertes Polysiloxan (ABIL B 8851)⁴⁾ | 1,0 Gew. % |
| Covarine W 9793, D&C Black 2 ²⁾ | 20,0 Gew. % |
| Glycerin | 4,0 Gew. % |
| Propylenglycol | 8,0 Gew. % |
| Ethanol | 8,0 Gew. % |
| Kunststoffdispersion auf Acrylat-Copolymer-basis, Feststoffgehalt 35 Gew.% (Syntran PC 5112)³⁾ | 20,0 Gew. % |

### Beispiel 2:

| | |
|---|---|
| Blau-Graue Eyelinerflüssigkeit mit einer Viskosität von ca. 8 mPa s (Brookfield, 25°C) Wasser | 61,0 Gew. % |
| Propylenglykol | 12,0 Gew. % |
| Ethanol | 5,0 Gew. % |
| Polyethylen-Glycerin-Stearinsäure-Ester (CAS 68553-11-7) | 1,0 Gew. % |
| Phenoxyethanol (Konservierung) | 0,5 Gew. % |
| Covarine W 9793, D & C Black 2 ²⁾ | 10,0 Gew. % |
| CI. 42090 | 0,5 Gew. % |
| Styrol/Acrylat/Ammonium-Methacrylat- | |
| Copolymer-Dispersion mit einem Gesamtfeststoffgehalt 41 Gew.% (Syntran 5760) ³⁾ | 10,0 Gew. % |

### Beispiel 3:

| | |
|---|---|
| Rotbraune Eyebrow Flüssigkeit mit einer Viskosität von ca. 15 mPa s (Brookfield, 25°C) | |
| Wasser | 53,28 Gew. % |
| 1,3 Butandiol | 12,0 Gew. % |
| Propanol | 3,0 Gew. % |
| Phenoxyethanol | 0,5 Gew. % |
| Polyethermodifiziertes Polysiloxan (ABIL B 8843)⁴⁾ | 3,0 Gew. % |
| Kunststoffdispersion auf Acrylat-Copolymer-basis, Feststoffgehalt 35 Gew.% (Syntran PC 5112)³⁾ | 25,0 Gew. % |
| C.I. 16035 (Rotfarbstoff) | 3,0 Gew. % |
| C.I 42090 (Blaufarbstoff) | 0,22 Gew. % |

Weitere Angaben zu den Beispielen:
1) dem. = demineralisiert
2) Sensient Cosmetic Technologies LCW, F-95310 Saint Ouen L'Aumone
3) Interpolymer SA'RL, F-67162 Wissembourg
4) Degussa, DE

## Patentansprüche

1. Wässrige kosmetische Zubereitung zur Färbung der Augenlider und der Augenbrauen, die
- 3 Gew.% bis 35 Gew.% einer wässrigen Kunststoffdispersion auf Acrylat-Copolymerbasis mit einem Feststoffgehalt von 30 Gew. % bis 50 Gew.%,
- 1 Gew.% bis 20 Gew. % wenigstens eines Alkohols aus der Gruppe Ethanol, Propanol,
- 2 Gew.% bis 15 Gew.% wenigstens eines Feuchthaltemittels,
- 0,2 Gew.% bis 5 Gew.% eines Polyoxyethylen-glycerinfettsäureesters und/oder eines polyethermodifizierten Polysiloxans, und
- ein Farbmittel
enthält, und die eine Viskosität von weniger als 50 mPas (Brookfield, 25°C) aufweist.

2. Zubereitung nach Anspruch 1,
**gekennzeichnet durch**
wenigstens ein Feuchthaltemittel aus der Gruppe 1,3-Butandiol, Propylenglykol und Glycerin.

3. Zubereitung nach Anspruch 2,
**dadurch gekennzeichnet,**
**dass** Glycerin stets in Mischung mit 1,3-Butandiol oder Propylenglykol vorhanden ist.

4. Zubereitung nach einem der vorhergehenden Ansprüche,
**gekennzeichnet durch**
einen Anteil an Farbmittel von 0,5 Gew.% bis 30 Gew.%.

5. Zubereitung nach Anspruch 4,
**dadurch gekennzeichnet,**
**dass** als Farbmittel Pigmente mit einer Korngrößenverteilung D90% < 10 µm enthalten sind.

6. Zubereitung nach einem der vorhergehenden Ansprüche,
**dadurch gekennzeichnet,**
**dass** max. 5 Gew.% Additive enthalten sind.

7. Zubereitung nach einem der vorhergehenden Ansprüche,
**dadurch gekennzeichnet,**
**dass** sie 30 Gew.% bis 70 Gew.% Wasser enthält.

## Claims

1. Aqueous cosmetic preparation for colouring eyelids and eyebrows, comprising
- 3% by weight to 35% by weight of an aqueous, acrylate copolymer-based plastics dispersion having a solids content of 30% by weight to 50% by weight,
- 1% by weight to 20% by weight of at least one alcohol selected from the group consisting of ethanol and propanol,
- 2% by weight to 15% by weight of at least one moisture-retaining agent,
- 0.2% by weight to 5% by weight of a polyoxyethylene glycerol fatty acid ester and/or of a polyether-modified polysiloxane, and
- a colorant,
and having a viscosity of less than 50 mPas (Brookfield, 25°C).

2. Preparation according to Claim 1, **characterized by** at least one moisture-retaining agent selected from the group consisting of 1,3-butanediol, propylene glycol and glycerol.

3. Preparation according to Claim 2, **characterized in that** glycerol is always present in admixture with 1,3-butanediol or propylene glycol.

4. Preparation according to any preceding claim, **characterized by** a colorant proportion of 0.5% by weight to 30% by weight.

5. Preparation according to Claim 4, **characterized in that** pigments having a particle size distribution D90% < 10 µm are present as colorant.

6. Preparation according to any preceding claim, **characterized in that** a maximum of 5% by weight of additives is included.

7. Preparation according to any preceding claim, **characterized in that** it contains 30% by weight to 70% by weight of water.

## Revendications

1. Préparation cosmétique aqueuse pour la coloration des paupières et des cils, qui comprend
- de 3 % en poids à 35 % en poids d'une dispersion aqueuse de matière plastique à base de copolymère d'acrylate ayant une teneur en matière solide de 30 % en poids à 50 % en poids,
- de 1 % en poids à 20 % en poids d'au moins un alcool du groupe éthanol, propanol,
- de 2 % en poids à 15 % en poids d'au moins un agent maintenant l'humidité;
- de 0,2 % en poids à 5 % en poids d'un ester d'acide gras de glycérine polyoxyéthyléné et/ou d'un polysiloxane modifié par un polyéther, et
- un colorant
et qui a une viscosité inférieure à 50 mPas (Brookfield, 25°C).

2. Préparation suivant la revendication 1,
**caractérisée par**
au moins un agent maintenant l'humidité du groupe 1,3 butanediol, propylèneglycol et glycérine.

3. Préparation suivant la revendication 2,
**caractérisée**
**en ce qu'**il y a de la glycérine toujours en mélange avec du 1,3 butanediol ou du propylèneglycol.

4. Préparation suivant l'une des revendications précédentes,
**caractérisée par**
une proportion de colorant de 0,5 % en poids à 30 % en poids.

5. Préparation suivant la revendication 4,
**caractérisée**
**en ce que**
**en ce qu'**elle contient comme colorant des pigments en une répartition granulométrique D90% < 10 µm.

6. Préparation suivant l'une des revendications précédentes,
**caractérisée**
**en ce qu'**elle contient au maximum 5 % en poids d'additifs.

7. Préparation suivant l'une des revendications précédentes,
**caractérisée**
**en ce qu'**elle contient de 30 % en poids à 70 % en poids d'eau.
